(19) Europäisches Patentamt
European Patent Office
Office européen des brevets

(11) Veröffentlichungsnummer: **0 012 216**
**B1**

# (12) EUROPÄISCHE PATENTSCHRIFT

(45) Veröffentlichungstag der Patentschrift: **26.08.81**

(51) Int. Cl.³: **C 07 D 231/12,** C 07 D 231/14, C 07 D 231/18

(21) Anmeldenummer: **79104325.0**

(22) Anmeldetag: **06.11.79**

## (54) Verfahren zur Herstellung weitgehend reiner Pyrazolverbindungen.

(30) Priorität: **15.11.78 DE 2849442**

(43) Veröffentlichungstag der Anmeldung: **25.06.80 Patentblatt 80/13**

(45) Bekanntmachung des Hinweises auf die Patenterteilung: **26.08.81 Patentblatt 81/34**

(84) Benannte Vertragsstaaten: **AT BE CH DE FR GB IT LU NL SE**

(56) Entgegenhaltungen: **DE-A-2 704 281**

(73) Patentinhaber: **BASF Aktiengesellschaft, Carl-Bosch-Strasse 38, D-6700 Ludwigshafen (DE)**

(72) Erfinder: **Linhart, Friedrich, Dr., Leisberg 61, D-6900 Heidelberg (DE)**
Erfinder: **Eicken, Karl, Dr., Waldstrasse 63, D-6706 Wachenheim (DE)**
Erfinder: **Girgensohn, Bjoern, Dr., Neckarpromenade 38, D-6800 Mannheim (DE)**
Erfinder: **Richarz, Winfried, Dr., Königsberger Strasse 5, D-6081 Stockstadt/Rh (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 012 216 B1

ACTORUM AG.

# Verfahren zur Herstellung weitgehend reiner Pyrazolverbindungen

Die vorliegende Erfindung betrifft ein Verfahren zur Herstellung weitgehend reiner Pyrazolverbindungen der allgemeinen Formel

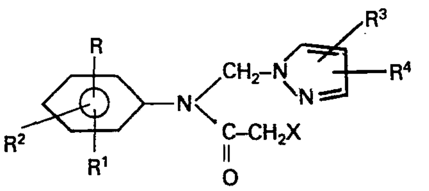

worin
R Wasserstoff, Halogen, Alkyl bis zu 5 C-Atomen, Alkoxy bis zu 3 C-Atomen, Perhalogenalkyl bis zu 3 C-Atomen oder Alkoxyalkyl bis zu 5 C-Atomen,
$R_1$ Wasserstoff, Halogen, Alkyl bis zu 5 C-Atomen, Alkoxy bis zu 3 C-Atomen, Perhalogenalkyl bis zu 3 C-Atomen oder Alkoxyalkyl bis zu 5 C-Atomen,
$R^2$ Wasserstoff, Halogen, Alkyl bis zu 5 C-Atomen, Alkoxy bis zu 3 C-Atomen, Perhalogenalkyl bis zu 3 C-Atomen oder Alkoxyalkyl bis zu 5 C-Atomen oder $R^2$ zusammen mit R eine orthoständig verknüpfte, gegebenenfalls durch Alkylgruppen mit bis zu 4 C-Atomen, substituierte Alkylenkette mit bis zu 6 C-Atomen bedeutet,
X Chlor oder Brom bedeutet,
$R^3$ und $R^4$ gleich oder verschieden sind und Wasserstoff, Alkyl, Alkoxy, Alkylthio, Carbalkoxy, Perfluoralkyl mit jeweils bis zu 4 C-Atomen, Halogen, Phenyl, Cyan oder Carboxy bedeuten, aus den entsprechenden Rohprodukten, beispielsweise wie sie bei der technischen Herstellung der Pyrazolverbindungen anfallen oder wie sie in verunreinigten Herstellungschargen der Pyrazolverbindungen vorliegen, falls der Herstellungsvorgang nicht ordnungsgemäss abgelaufen sein sollte oder wie sie bei einem vereinfachten Verfahren zur Herstellung der Pyrazolverbindungen ohne Reinigung der Zwischenprodukte anfallen durch Behandlung der Rohprodukte mit wässrigen Lösungen starker Säuren und Gewinnung der reinen Produkte aus den wässrigen Lösungen.

Die Herstellung dieser Pyrazolverbindungen ist aus den DE-A-26 48 008 und DE-A-27 04 281 bekannt. Nach den dort beschriebenen Verfahren fallen die Produkte in einem organischen Lösungsmittel, z.B. Chloroform, Toluol, Petroläther, Essigester, Benzin gelöst oder suspendiert an und müssen dann von diesem Lösungsmittel abgetrennt werden, beispielsweise durch Abdampfen des Lösungsmittels oder Filtration. Diese Methoden sind im Labor sehr gut zu handhaben und liefern auch sehr saubere Produkte.

Will man diese Laborverfahren in einem grösseren Massstab, z.B. einen Technikums- oder Produktionsmassstab, übertragen, so treten unerwartete Schwierigkeiten auf, von denen einige im folgenden genannt werden:

Liegt das Produkt in organischer Lösung vor, so muss das Lösungsmittel, gegebenenfalls unter vermindertem Druck, abgedampft werden. Dabei kristallisiert das Produkt bevorzugt an den Wandungen des verwendeten Eindampfgefässes, z.B. eines Kessels aus oder backt dort fest. Es ist bekannt, dass es ausserordentlich schwierig ist, aus einem Reaktionskessel mit den üblichen kleinen Zu- und Ablauföffnungen ein festes, insbesondere ein festgebackenes Produkt zu entnehmen. Dazu muss der verwendete Apparat nämlich fachmännisch auseinander gebaut werden, was einen grossen Zeit- und Personalaufwand bedeutet. Das zu diesem Zweck heranzuziehende Fachpersonal (Schlosser) ist normalerweise aber nicht im Umgang mit Chemikalien und im Beachten chemischer Sicherheitsvorschriften geschult und könnte durch Einatmen von oder Kontakt mit anhaftendem Restlösungsmittel oder Produktstaub Schaden erleiden.

Gelingt es, das Produkt in einem organischen Lösungsmittel suspendiert zu erhalten, so muss man letzteres durch Filtration entfernen. Je nach Giftigkeit und Explosivität des Lösungsmittels muss man dabei mehr oder weniger aufwendige Sicherheitsvorkehrungen treffen, die wiederum einen hohen Zeit- und Kostenaufwand bedeuten. Häufig gehen mit dem Filtrat auch beträchtliche gelöste Produktmengen verloren.

So kann man z.B. das in einem organischen Lösungsmittel gelöste Produkt ausfällen durch Zugabe eines zweiten, mit dem ersten mischbaren Lösungsmittels, in dem das Produkt aber schwerlöslich ist. Je nach Menge des Fällungslösungsmittels fällt aber mehr oder weniger Produkt aus, wobei seine Reinheit wiederum von der Menge des Fällungsmittels abhängt.

Man kann die Produkte in bekannter Weise auch isolieren, indem man sie aus ihrer Lösung in einem wasserfreien Lösungsmittel, in dem ihre Salze unlöslich sind, durch Zugabe von überschüssiger wasserfreier Säure, z.B. durch Einleiten von gasförmigen Chlorwasserstoff, in Form ihrer Salze ausfällt. Aber auch dann muss man unter Beachtung aller Sicherheitsvorschriften das säurehaltige organische Lösungsmittel vom Salz absaugen, wobei erschwerend hinzukommt, dass die überschüssige Säure sowohl dem Produkt anhaften kann und umweltbelästigend und korrosiv wirken kann, als auch im Filtrat vorliegt und bei der Aufarbeitung oder Vernichtung des Lösungsmittels Schwierigkeiten bereitet. Häufig finden beim Versetzen von organischen Lösungsmitteln mit konzentrierten wasserfreien Säuren, z.B. Chlorwasserstoff, Schwefelsäure oder Salpetersäure, auch unerwünschte oder gar gefährliche Nebenreaktionen statt. Bei der bekannten Ausfällung der Produkte mit Säure kommt als Nachteil hinzu, dass nicht nur das gewünschte Produkt selbst, sondern auch sämtliche anderen zur Salzbildung befähigten Nebenprodukte mit ausgefällt werden. Ferner ist zu berücksichtigen, dass die als Salze gefällten Produkte in einem zusätzlichen Arbeitsgang wieder in die entsprechenden freien Basen umgewandelt werden müssen. Dies ist unbedingt erforderlich, da die Salze in feuchter Umgebung, z.B. an

der Luft, hydrolysieren und dabei ätzende und korrosive Säure freisetzen.

Ein weiteres Hindernis bei der Übertragung der bekannten und an sich sehr guten Laborverfahren in den Technikums- oder Produktionsmassstab besteht darin, dass man für Arbeiten im grossen Massstab häufig nicht so saubere Reagenzien und Lösungsmittel sowie kein chemisch so gut geschultes Personal zur Verfügung hat wie im Labor. Dadurch kommt es, dass die anfallenden Produkte meistens mit Nebenprodukten verunreinigt sind. Die Entfernung dieser Nebenprodukte ist fast immer sehr umständlich und gelingt nur selten vollständig. Zum Beispiel kann man einige der bekannten Produkte aus Cyclohexan umkristallisieren. Dabei lassen sich jedoch die häufigsten Verunreinigungen, nämlich Verbindungen der allgemeinen Formel

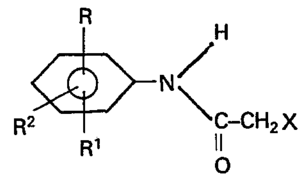

in der R, $R^1$, $R^2$ und X die oben beschriebene Bedeutung haben, nur unvollkommen aus den Pyrazolverbindungen entfernen, da sie in kaltem Cyclohexan ebenfalls schwerlöslich sind. Ausserdem gehen beim Umkristallisieren grosse Mengen der Pyrazolverbindungen verloren. Bei der Isolierung der umkristallisierten Substanzen aus organischen Lösungsmitteln in grossem Massstab treten natürlich auch wieder die oben beschriebenen Probleme auf.

Es wurde daher ein Verfahren gesucht, mit dessen Hilfe es gelingt, selbst aus sehr grossen Technikums- und Produktionsansätzen die Pyrazolverbindung auf einfache Art in hoher Reinheit zu isolieren und mit dessen Hilfe man auch verunreinigte Chargen der Pyrazolverbindung leicht reinigen kann.

Bei den Pyrazolverbindungen handelt es sich um sogenannte Aminale, da sie 2 Stickstoffatome an eine Methylengruppe gebunden enthalten. Es ist in der Fachwelt seit langem bekannt, dass sich ein Aminal mit starker Säure in wässriger Lösung in eine Carbonylverbindung und zwei Stickstoffverbindungen spalten lässt (P.A.S. Smith, Open-Chain Nitrogen Compounds, W.A. Benjamin, Inc., New York, Amsterdam 1965, Vol. I, S. 322).

Es hat sich überraschend gezeigt, dass die Pyrazolverbindungen, obwohl sie Aminalstrukturen besitzen, gegen starke wässrige Säuren stabil sind und sich in 30- bis 90gew.-%igen wässrigen starken anorganischen Säuren sogar unzersetzt in Form ihrer Salze lösen.

Da die Pyrazolverbindungen aber nur sehr schwache Basen sind, bleiben sie in verdünnten wässrigen Säuren ungelöst.

Das erfindungsgemässe Verfahren besteht demnach darin, dass man die Rohprodukte mit konzentrierten wässrigen Lösungen von starken Säuren behandelt, danach die wässrige Lösung abtrennt, mit Wasser verdünnt und das dabei ausfallende reine Produkt von der wässrigen Flüssigkeit abtrennt. Beispielsweise wird die Lösung des Rohproduktes in einem mit Wasser nicht oder nur wenig mischbaren organischen Lösungsmittel mit einer konzentrierten wässrigen Lösung einer starken Säure innig vermischt, die wässrige Phase abgetrennt und dann mit Wasser verdünnt, wobei das Produkt in reiner Form ausfällt und aus der wässrigen Suspension leicht abfiltriert und getrocknet werden kann.

Man kann beispielsweise aber auch kristallisiertes Rohprodukt oder verunreinigtes Rohprodukt in fester, feinverteilter Form mit einer konzentrierten wässrigen Säure innig vermischen, filtrieren und das Filtrat mit Wasser verdünnen und das dabei ausfallende Reinprodukt absaugen. Die Trocknung der wasserfeuchten reinen Produkte schafft keine technischen oder Sicherheitsprobleme.

Liegt das Rohprodukt in Form einer Lösung in einem organischen Lösungsmittel vor, in dem sich ein Teil der Verunreinigungen nur schlecht löst, z.B. in einem offenkettigen oder cyclischen Paraffinkohlenwasserstoff wie Hexan, Heptan, Oktan, Cyclohexan, Petroläther, Ligroin, Benzin und ähnliche, so empfiehlt es sich, die Mischung mit der wässrigen Säure vor der Phasentrennung zu filtrieren, um die Phasen sauber trennen zu können. Als Lösungsmittel für das Rohprodukt eignen sich ausser den bereits genannten alle Lösungsmittel, die mit wässrigen Säuren nicht oder nur sehr wenig mischbar sind, z.B. aromatische Kohlenwasserstoffe wie Benzol, Toluol, Xylol, Chlorbenzol, Dichlorbenzol, Chlortoluol, höhere Alkylbenzole; aliphatische Chlorkohlenwasserstoffe wie Dichlormethan, Chloroform, Dichloräthan, Tetrachlormethan; Äther wie Diäthyläther, Diisopropyläther, und andere. Man kann auch Ester, Amide, Nitrile und ähnliche Verbindungen als Lösungsmittel verwenden, sofern diese Lösungsmittel genügend stabil gegen wässrige Säuren sind oder sofern man schnell genug arbeitet, um eine nennenswerte Hydrolyse der Lösungsmittel zu verhindern. Nicht in Frage kommen basische Lösungsmittel, die mit den verwendeten Säuren Salze bilden.

Erfindungsgemäss verwendbare starke anorganische Säuren sind z.B. Salzsäure, Bromwasserstoffsäure, Perchlorsäure, Schwefelsäure, Salpetersäure, Phosphorsäure. Die verwendeten konzentrierten Lösungen der Säuren enthalten vorzugsweise zwischen 20 und 65 Gew.-% Wasser. Besonders geeignet sind 32- bis 38%ige Salzsäure oder 40- bis 80%ige Schwefelsäure. Mehr als 50%ige Salpetersäure eignet sich wegen ihrer Oxidationswirkung schlecht.

Die Menge der verwendeten Säure muss so bemessen sein, dass sämtliche in dem organischen Lösungmittel gelösten oder im festen Rückstand befindlichen basischen Substanzen in ihr Salz übergeführt werden. Dazu ist mindestens die der theoretisch berechneten Menge der Pyrazolverbindung äquivalente Menge an Säure erforderlich. Es empfiehlt sich jedoch, einen Säureüberschuss zu verwenden, der zwischen 10 und 1000%

liegen kann. Als besonders günstig hat sich die Verwendung eines 2- bis 5-fachen molaren Überschusses erwiesen. Falls trotz Verwendung eines solchen Überschusses noch ein Teil des gewünschten Produktes im organischen Lösungsmittel bzw. im festen Rückstand zurückbleibt, dann kann man das Rohprodukt natürlich auch ein zweites Mal oder noch mehrmals mit konzentrierter wässriger Säure behandeln. Selbstverständlich kann man die Pyrazolverbindungen auch kontinuierlich aus ihrer Lösung oder einem sie enthaltenden festen Rückstand mit Säure extrahieren.

Die Temperatur spielt für die Extraktion keine grosse Rolle, wenn man davon absieht, dass bei zu hoher Temperatur Zersetzungen und schlechte Phasentrennungen eintreten können oder zuviel unpolare Nebenprodukte in die Wasserphase übergehen können und dass bei zu tiefen Temperaturen sowohl Produkt als auch Lösungsmittel und Wasser durch Auskristallisation Schwierigkeiten bereiten können. Temperaturen zwischen 0 und 50 °C haben sich als günstig erwiesen, insbesondere Umgebungstemperatur. Die Menge des zur Verdünnung verwendeten Wassers sollte so bemessen sein, dass das gewünschte Produkt möglichst quantitativ ausfällt. Man kann, bezogen auf die Säuremenge, die 2- bis 100-fache Menge an Wasser verwenden, doch hat es sich aus praktischen Gründen als am günstigsten erwiesen, den Säureextrakt auf das 5- bis 10-fache zu verdünnen. Die Temperatur bei der Verdünnung spielt keine wesentliche Rolle, doch sollte man beachten, dass einige der auszufällenden Substanzen einen niedrigen Schmelzpunkt besitzen und deshalb bei zu hoher Temperatur als Öle, die evtl. Verunreinigungen einschliessen, ausfallen können, und dass bei Erhöhung der Temperatur die Widerstandsfähigkeit der Pyrazolverbindungen gegen wässrige Säure abnehmen kann. Darum verdünnt man im allgemeinen bei 0 °C bis 50 °C, am einfachsten bei der Temperatur der Umgebung. Zur Verdünnung kann man das Wasser zur Säure geben, doch erweist es sich im allgemeinen als besser, die Säure unter gutem Rühren zum Wasser zulaufen zu lassen. Dabei fällt dann nur die gewünschte reine Pyrazolverbindung aus, während alle anderen gelösten Substanzen in Lösung bleiben.

Das reine Produkt kann dann beispielsweise über eine grosse Nutsche abgesaugt und mit Wasser gewaschen werden und wird dabei so geruchsneutral, dass es sogar von Hand aus der Nutsche ausgeschaufelt und gegebenenfalls in eine Trockenapparatur gefüllt werden kann.

Soll das Produkt aber zur Weiterverarbeitung wieder in einem organischen Lösungsmittel gelöst werden, so kann man es natürlich direkt nach dem Ausfällen aus der sauren verdünnten wässrigen Lösung mit dem gewünschten Lösungsmittel extrahieren, sofern dieses Lösungsmittel mit Wasser nicht mischbar ist.

Das erfindungsgemässe Verfahren beruht in chemischer Hinsicht darauf, dass sich in einer ersten Reinigungsstufe die schwach basischen, überraschenderweise säurestabilen Pyrazolverbindungen zusammen mit anderen polaren oder zur Salzbildung befähigten Verbindungen der Reaktionsmischung in der konzentrierten Säure lösen, während die unpolaren Substanzen entweder im organischen Lösungsmittel zurückbleiben, oder, falls man auf letzteres verzichtet, als fester Rückstand abfiltriert werden. Die zweite wesentliche chemische Grundlage des Verfahrens besteht darin, dass die stark sauren gelösten Säureadditionssalze der Pyrazolverbindungen in einer zweiten Reinigungsstufe bereits in verdünnter Säurelösung wieder in Säure und Pyrazolverbindung zerfallen, während alle anderen polaren und salzartigen Verbindungen in der beim Versetzen mit Wasser entstandenen verdünnten Säure gelöst bleiben. Durch diese zweifache Reinigung kommt der hohe Reinheitsgrad der Endprodukte zustande.

Das erfindungsgemässe Verfahren eignet sich ausgezeichnet zur Isolierung der Pyrazolverbindungen aus ihren rohen Reaktionslösungen und lässt sich auch im Technikums- und Produktionsmassstab sehr leicht handhaben. Das erfindungsgemässe Verfahren lässt sich auch leicht zur Reinigung grosser verunreinigter Mengen von Pyrazolverbindungen einsetzen. Mit Hilfe des Verfahrens lassen sich selbst aus Fehlchargen, bei denen die Herstellungsreaktion der Pyrazolverbindungen nicht ordnungsgemäss verlaufen ist, die häufig nur in geringen Mengen vorliegenden wertvollen Pyrazolverbindungen einwandfrei isolieren.

Es gelingt darüber hinaus, auch solche Pyrazolverbindungen zu reinigen, die nach einem vereinfachten Verfahren hergestellt worden sind. Es ist bekannt, Pyrazolverbindungen dadurch herzustellen, dass man Verbindungen der allgemeinen Formel

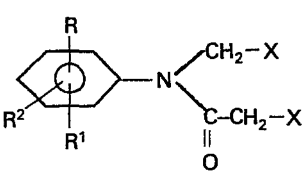

in der R, $R^1$, $R^2$ und X die oben angegebene Bedeutung haben, gegebenenfalls in Gegenwart eines Halogenwasserstoff-bindenden Mittels und in Gegenwart eines inerten Lösungsmittels, mit einem Pyrazol der allgemeinen Formel

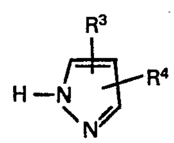

in der $R^3$ und $R^4$ die oben angegebene Bedeutung haben, umsetzt. Es hat sich nun herausgestellt, dass die Handhabung grösserer Menge von N-Halogenacetyl-N-halogenmethylanilinen äusserst unangenehm und nicht ungefährlich ist. Die Substanzen sind im allgemeinen fest und lassen sich nicht pumpen und durch geschlossene Leitungen transportieren. Ihre Dämpfe wirken stark schleimhautreizend und der Kontakt mit ihrem Staub kann Hautausschläge verursachen. Wie der Fachmann schon allein aufgrund der chemischen Formel erkennt, können diese Verbindungen mit Wasser oder an feuchter Luft, an Schleimhäuten oder feuchter Haut die giftigen Gase Formaldehyd und Chlorwasserstoff abspalten.

Aus diesen Gründen lassen sich grössere Mengen dieser Verbindungen nur unter Anwendung aufwendiger Sicherheitsmassnahmen verarbeiten.

Für die Herstellung dieser Verbindungen sind mehrere Methoden bekannt. Eine einfache Methode (US-PS 3 637 847) besteht darin, dass man ein Azomethin der allgemeinen Formel

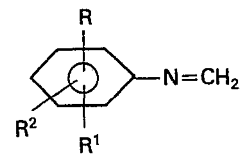

in der R, $R^1$ und $R^2$ die oben angegebene Bedeutung besitzen, mit einem Halogenoacetylhalogenid der allgemeinen Formel

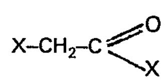

in welcher X die oben angegebene Bedeutung hat, umsetzt. Die Azomethine werden nach bekannten Verfahren aus Formaldehyd und Anilinen der allgemeinen Formel

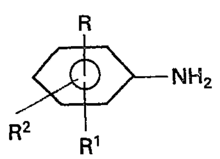

in welcher R, $R^1$ und $R^2$ die oben angegebenen Bedeutungen haben, synthetisiert, und vor ihrer Verwendung zur Reinigung destilliert. Es ist jedoch aus der DE-AS 17 93 811 bekannt, dass einige Azomethine, die als Vorprodukt zur Herstellung von Pyrazolverbindungen besonders interessant sind, z.B. jenes, in dem R und $R^1$ je eine zum Sickstoff ortho-ständige Methylgruppe und $R^2$ ein Wasserstoffatom bedeuten, nicht beständig sind und nicht zur Synthese anderer Verbindungen verwendet werden können.

Es wurde nun überrraschenderweise gefunden, dass man durch Umsetzung eines Anilins der allgemeinen Formel

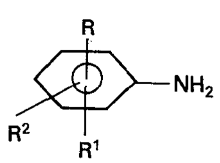

in der R, $R^1$ und $R^2$ die im Anspruch 1 angegebene Bedeutung haben, mit Paraformaldehyd in einem inerten organischen Lösungmittel, Abdestillieren von Wasser und überschüssigem Formaldehyd aus der Reaktionsmischung, Umsetzung von einem Halogenacetylhalogenid mit der Reaktionsmischung und weitere Umsetzung der Reaktionsmischung mit einem Pyrazol der allgemeinen Formel

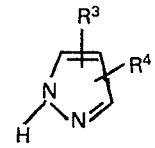

in welcher $R^3$ und $R^4$ die im Anspruch 1 angegebene Bedeutung haben, und einem Halogenwasserstoff-bindenden Mittel, die Pyrazolverbindungen in hoher Reinheit und guter Ausbeute erhält, ohne dass man die oben beschriebenen Zwischenprodukte isolieren und reinigen muss, wenn man die Pyrazolverbindungen nach dem erfindungsgemässen Verfahren aus ihren rohen Lösungen isoliert. Selbst Pyrazolverbindungen, die auf Azomethinen basieren, welche nach allgemeiner Erfahrung und nach der DE-AS 17 93 811 unbeständig sind, lassen sich leicht herstellen, wenn die betreffenden Azomethine nicht gereinigt, destilliert oder anderweitig isoliert, sondern in ihrer Rohlösung weiterverarbeitet werden.

Das Verfahren besteht darin, dass man ein Anilin in an sich bekannter Weise mit Paraformaldehyd zu einem Azomethin umsetzt, Wasser und Formaldehyd abdestilliert, die rohe Lösung des Azomethins im gleichen oder einem anderen Gefäss mit einem Halogenacetylhalogenid vermischt, die Mischung mit einem Pyrazol und mit einem Halogenwasserstoff-bindenden Mittel versetzt und die Mischung erfindungsgemäss mit einer konzentrierten wässrigen Säure behandelt, die Säure abtrennt und mit Wasser verdünnt, wobei das Endprodukt sauber und in guter Ausbeute anfällt. Man kann die Reaktionsmischung vor der Behandlung mit Säure natürlich auch mit Wasser waschen. Eine Variante des Verfahrens besteht darin, dass man als Halogenwasserstoff-bindendes Mittel eine wässrige Base gegebenenfalls in Anwesenheit eines sogenannten Phasen-Transfer-Katalysators verwendet, wobei man dann die Reaktionsmischung natürlich erst nach Abtrennung der wässrigen Phase mit der konzentrierten Säure behandelt.

Die Vorteile dieses Verfahrens bestehen darin, dass man erstens auch unbeständige Azomethine verwenden kann und insbesondere zweitens das oben erwähnte problematische Zwischenprodukt nicht zu isolieren braucht. Da die gesamte Reaktionsfolge in ein oder zwei geschlossenen Apparaten durchgeführt werden kann, ohne dass zwischenzeitlich isoliert oder aufgearbeitet werden muss, kommt das Bedienungspersonal nicht mit den gefährlichen Zwischenprodukten in Berührung. Wenn man bei mehrstufigen Reaktionen die Produkte der einzelnen Reaktionsschritte nicht isoliert und reinigt, so reichern sich, wie dem Fachmann geläufig ist, zum Schluss die Verunreinigungen so stark an, dass die Isolierung und Reinigung des Endproduktes schwierig ist. Dies trifft im vorliegenden Falle jedoch nicht zu, da man das Rohprodukt nach dem erfindungsgemässen Verfahren leicht reinigen kann.

Das erfindungsgemässe Verfahren zur Reinigung von Pyrazolverbindungen eignet sich selbstverständlich auch zur Reinigung von Rohlösungen, die auf anderem Weg z.B. nach DE-OS 27 04 281, erhalten wurden und führt auch dort zu enormen Arbeitserleichterungen.

Die folgenden Beispiele zeigen die Einfachheit und Zuverlässigkeit des erfindungsgemässen Verfahrens.

Beispiel 1

5100 Teile (Gewichtsteile) N-Chlormethyl-2,6-dimethylchloracetanilid und 11000 Teile Toluol werden mit 1900 Teilen 4-Methylpyrazol versetzt und dann 3 Stunden bei 40 bis 60 °C gerührt. Man gibt 2130 Teile Triäthylamin zu, rührt 3 Stunden bei 60 °C und über Nacht bei Raumtemperatur (20 °C). Die Reaktionsmischung wird zweimal mit 10000 Teilen Wasser gewaschen und einmal mit 12000 Teilen 37%iger (Gew.-%) Salzsäure extrahiert. Den Extrakt lässt man unter Rühren zu 60000 Teilen Wasser laufen und saugt den Niederschlag ab. Nach dem Trocknen erhält man 4750 Teile 95%iges N-(4-Methylpyrazolyl-(1)-methyl)-2,6-dimethylchloracetanilid vom Schmelzpunkt 98–100 °C.

Beispiel 2

1300 Teile N-Chlormethyl-2-äthyl-6-methylchloracetanilid und 3000 Teile Toluol werden mit 540 Teilen 4-Methoxypyrazol versetzt. Man hält 4 Stunden bei 60 °C, gibt 500 Teile Triäthylamin zu, rührt 4 Stunden bei 60 °C und über Nacht bei Raumtemperatur. Man verrührt mit 2000 Teilen Wasser und saugt die dabei ausfallenden Kristalle ab, die nach dem Waschen mit Wasser und Trocknen 750 Teile reines N-(4-Methoxypyrazolyl-(1)-methyl)-6-äthyl-2-methylchloracetanilid vom Schmelzpunkt 96–97 °C ergeben. Die organische Phase des Filtrats wird abgetrennt und einmal mit 950 Teilen 37%iger Salzsäure extrahiert. Die beim Eintropfen des Extrakts in 6000 Teile Wasser ausfallenden Kristalle ergeben nach dem Absaugen und Trocknen nochmals 660 Teile 98%iges N-(4-Methoxypyrazolyl-(1)-2-äthyl-6-methylchloracetanilid vom Schmelzpunkt 92–93 °C.

Beispiel 3

a) 14 Teile 94%iges N-(Pyrazolyl-(1)-methyl)-2,6-dimethylchloracetanilid vom Schmelzpunkt 72–75 °C, dessen Gehalt protonenresonanzspektroskopisch bestimmt wurde, werden in 140 Teilen Toluol gelöst und mit 40 Teilen 60%iger Schwefelsäure einmal ausgeschüttelt. Den schwefelsauren Extrakt lässt man unter gutem Rühren zu 240 Teilen Wasser laufen, saugt die ausgefallenen Kristalle ab, wäscht mit Wasser und trocknet. Man erhält 11 Teile 100%iges N-(Pyrazolyl-(1)-methyl)-2,6-dimethylchloracetanilid vom Schmelzpunkt 83 °C.

b) 14 Teile 94%iges N-(Pyrazolyl-(1)-methyl)-2,6-dimethylchloracetanilid werden in 140 Teilen Toluol gelöst und zweimal mit je 40 Teilen 50%iger Schwefelsäure extrahiert. Die vereinigten schwefelsauren Extrakte werden mit Wasser hydrolysiert. Der ausgefallene Niederschlag wird abgesaugt, mit Wasser gewaschen und getrocknet, wobei man 13 Teile 100%iges Produkt erhält mit dem Schmelzpunkt 83 °C.

Beispiel 4

a) 100 Teile einer schwarzbraunen Schmiere, welche bei einer nicht ordnungsgemäss verlaufenen Herstellung von N-(Pyrazolyl-(1)-methyl)-2,6-dimethylchloracetanilid entstanden waren, werden mit 180 Teilen 37%iger Salzsäure eine halbe Stunde gut verrührt. Nach dem Absaugen wird das Filtrat mit 50 Teilen Toluol extrahiert und die Säure in 1000 Gewichtsteile Wasser getropft. Man erhält nach dem Absaugen und Trocknen 60 Teile 93%iges N-(Pyrazolyl-(1)-methyl)-2,6-dimethylchloracetanilid vom Schmelzpunkt 77 °C.

b) 100 Teile der unter a) genannten Schmiere werden mit 150 Teilen Toluol gut verrührt und filtriert. Das Filtrat wird einmal mit 180 Teilen 37%iger Salzsäure ausgeschüttelt. Die Salzsäurephase tropft man in 900 Teile Wasser, saugt ab und trocknet. Man erhält 54 Teile 97%iges N-(Pyrazolyl-(1)-methyl)-2,6-dimethylchloracetanilid vom Schmelzpunkt 78 °C.

c) 100 Teile der unter a) genannten Schmiere werden in 180 Teilen Methylenchlorid gelöst, einmal mit 180 Teilen und danach mit 60 Teilen 37%iger Salzsäure extrahiert. Die vereinigten salzsauren Extrakte werden zur Entfernung von Methylenchlorid einmal mit 50 Teilen Toluol gewaschen. Man hydrolysiert wie unter a) und b) beschrieben und erhält 56,5 Teile mehr als 99%iges N-(Pyrazolyl-(1)-methyl)-2,6-dimethylchloracetanilid vom Schmelzpunkt 83 °C.

Beispiel 5

Zu einer Lösung von 202 Teilen Bromacetylbromid in 150 Teilen Ligroin tropft man eine rohe Lösung von 133 Teilen N-(2,6-Dimethylphenyl)-methylenimin in einem Toluol-Cyclohexan-Gemisch. Da statt des gewünschten kristallinen N-Brommethyl-2,6-dimethyl-bromacetanilids nur ein schwer isolierbares Öl ausfällt, gibt man 450 Teile Toluol und 75 Teile Pyrazol zu, erhitzt 2 Stunden auf 60 °C, tropft 110 Teile Triäthylamin zu, erhitzt auf 60 °C, wäscht mit Wasser, danach mit 5%iger Salzsäure und danach nochmal mit Wasser und dampft ein, wobei man 248 Teile eines zähen Öles erhält. Dieses wird in 500 Teilen Toluol gelöst und mit 250 Teilen 37%iger Salzsäure extrahiert. Der Extrakt wird mit 1500 Teilen Wasser hydrolysiert, wobei man 190 Teile N-(Pyrazolyl-(1)-methyl)-2,6-dimethylbromacetanilid vom Schmelzpunkt 86 °C erhält.

Beispiel 6

Aus einer Mischung von 10890 Teilen 2,6-Dimethylanilin und 4050 Teilen Paraformaldehyd werden mit 50000 Teilen eines Toluol-Cyclohexan-Gemisches in an sich bekannter Weise 1620 Teile Wasser durch Sieden am Rückfluss entfernt. Nach dem schnellen Abkühlen auf Raumtemperatur setzt man die so erhaltene Lösung von N-(2,6-Dimethylphenyl)-methylenimin mit ihren Verunreinigungen in an sich bekannter Weise mit 10800 Teilen Chloracetylchlorid in 10000 Teilen Toluol um. Man gibt danach 6800 Teile Pyrazol zu, rührt 5 Stunden bei 50 °C, gibt dann 10000 Teile Triäthylamin zu, rührt 5 Stunden bei 50 °C und über Nacht bei Raumtemperatur. Dann wird die Reaktionsmischung mit 30000 Teilen 5%iger Salzsäure gewaschen und die abgetrennte organische Phase mit 30000 Teilen 37%iger Salzsäure extrahiert. Der Extrakt wird in 180000 Teile Wasser eingerührt. Der ausgefallene Niederschlag wird abgesaugt, mit Wasser gewaschen und getrocknet.

Man erhält 17840 Teile 96%iges N-(Pyrazolyl-(1)-methyl)-2,6-dimethylchloracetanilid vom Schmelzpunkt 78 °C.

Beispiel 7

121 Teile einer stark verunreinigten Charge von N-(Pyrazolyl-(1)-methyl)-2-äthyl-6-methylbromacetanilid werden in 300 Teilen Toluol gelöst, mit 200 Teilen 37%iger Salzsäure extrahiert und der Extrakt mit Wasser hydrolysiert. Man erhält 67 Teile des reinen Produktes vom Schmelzpunkt 71 bis 73 °C.

Beispiel 8

Aus 6050 Teilen 2,6-Dimethylanilin und 2400 Teilen Paraformaldehyd werden mit Hilfe eines Toluol-Cyclohexan-Gemisches 900 Teile Wasser wie in Beispiel 6 entfernt. Die erhaltene Lösung gibt man zu 6000 Teilen Chloracetylchlorid in 6000 Teilen Toluol, setzt nach Beendigung der Reaktion 3800 Teile Pyrazol zu, hält 4 Stunden bei 50 °C, gibt 5500 Teile Triäthylamin zu und erwärmt 6 Stunden auf 50 °C. Man wäscht mit verdünnter Salzsäure (5%) und saugt die Mischung von etwas Rückstand ab. Die Toluolphase des Filtrats wird zweimal mit je 13000 Teilen 60%iger Schwefelsäure extrahiert. Den Extrakt rührt man in 300000 Teile Wasser ein. Der ausfallende Niederschlag wird abgesaugt und getrocknet und ergibt 8800 Teile 98%iges N-(Pyrazolyl-(1)-methyl)-2,6-dimethyl-chloracetanilid vom Schmelzpunkt 81–83 °C.

Beispiel 9

Aus 121 Teilen 2,6-Dimethylanilin und 45 Teilen Paraformaldehyd in 250 Teilen eines Toluol-Cyclohexan-Gemisches werden wie in Beispiel 6 beschrieben 18 Teile Wasser entfernt. Nach dem schnellen Abkühlen auf Raumtemperatur lässt man die so erhaltene Lösung von N-(2,6-Dimethylphenyl)-methylenimin in an sich bekannter Weise mit 113 Teilen Chloracetylchlorid in 100 Teilen Toluol reagieren und rührt ½ Stunde bei 80 °C nach. Dann tropft man bei Raumtemperatur eine Mischung aus 44 Teilen NaOH, 75 Teilen Pyrazol und 6,8 Teilen einer Mischung aus 35% (Gew.-%) Dimethyldibenzylammoniumchlorid, 15% Trimethylbenzylammoniumchlorid, 40% Wasser und 10% Methanol in 200 Teilen Wasser zu und rührt 4 Stunden bei Raumtemperatur. Nach dem Abtrennen der organischen Phase von Wasser wäscht man diese zweimal mit 200 Teilen Wasser und extrahiert sie anschliessend einmal mit 350 Teilen und danach mit 175 Teilen 55%iger Schwefelsäure. Die vereinigten schwefelsauren Extrakte werden in 3000 Teile Wasser eingerührt, der ausgefallene Niederschlag wird abgesaugt, mit Wasser gewaschen und im Vakuum bei 50 °C getrocknet.

Man erhält 210 Teile 98%iges N-(Pyrazolyl-(1)-methyl)-2,6-dimethylchloracetanilid vom Schmelzpunkt 82 °C.

**Patentansprüche**

1. Verfahren zur Herstellung weitgehend reiner Pyrazolverbindungen der allgemeinen Formel

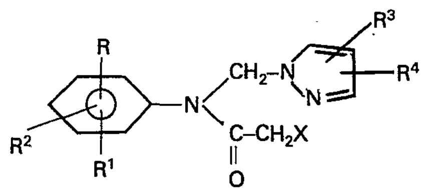

(I)

worin

R Wasserstoff, Halogen, Alkyl bis zu 5 C-Atomen, Alkoxy bis zu 3 C-Atomen, Perhalogenalkyl bis zu 3 C-Atomen oder Alkoxyalkyl bis zu 5 C-Atomen,

$R^1$ Wasserstoff, Halogen, Alkyl bis zu 5 C-Atomen, Alkoxy bis zu 3 C-Atomen, Perhalogenalkyl bis zu 3 C-Atomen oder Alkoxyalkyl bis zu 5 C-Atomen,

$R^2$ Wasserstoff, Halogen, Alkyl bis zu 5 C-Atomen, Alkoxy bis zu 3 C-Atomen, Perhalogenalkyl bis zu 3 C-Atomen oder Alkoxyalkyl bis zu 5 C-Atomen oder $R^2$ zusammen mit R eine orthoständig verknüpfte, gegebenenfalls durch Alkylgruppen mit bis zu 4 C-Atomen, substituierte Alkylenkette mit bis zu 6 C-Atomen bedeutet,

X Chlor oder Brom bedeutet,

$R^3$ und $R^4$ gleich oder verschieden sind und Wasserstoff, Alkyl, Alkoxy, Alkylthio, Carbalkoxy, Perfluoralkyl mit jeweils bis zu 4 C-Atomen, Halogen, Phenyl, Cyan oder Carboxy bedeuten, aus den entsprechenden Rohprodukten, dadurch gekennzeichnet, dass man die Rohprodukte mit 30- bis 90gew.-%igen wässrigen Lösungen von starken anorganischen Säuren behandelt, danach die wässrige Lösung abtrennt, mit Wasser verdünnt und das dabei ausfallende reine Produkt von der wässrigen Flüssigkeit abtrennt.

2. Verfahren gemäss Anspruch 1, dadurch gekennzeichnet, dass man 32 bis 38%ige Salzsäure verwendet.

3. Verfahren gemäss Anspruch 1, dadurch gekennzeichnet, dass man 40 bis 80%ige Schwefelsäure verwendet.

4. Verfahren gemäss Anspruch 1, dadurch gekennzeichnet, dass man die ausgefallenen reinen Produkte mit einem für ihre Weiterverarbeitung geeigneten organischen Lösungsmittel aus der wässrigen Flüssigkeit extrahiert.

5. Verfahren gemäss Anspruch 1, dadurch gekennzeichnet, dass man Lösungen der Rohprodukte in mit Wasser nicht oder nur wenig mischbaren organischen Lösungmitteln als Ausgangsprodukte verwendet.

6. Verfahren gemäss Anspruch 1, dadurch gekennzeichnet, dass man die Rohprodukte in fester, feinverteilter Form als Ausgangsprodukte verwendet.

7. Verfahren gemäss Anspruch 1, dadurch gekennzeichnet, dass man als Rohprodukte solche Produkte verwendet, die man erhält durch Umsetzung eines substituierten Anilins der allgemeinen Formel

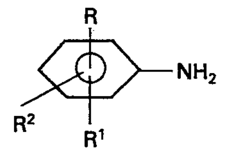

in der R, $R^1$ und $R^2$ die im Anspruch 1 angegebene Bedeutung haben, mit Paraformaldehyd in einem inerten organischen Lösungsmittel, Abdestillieren von Wasser und überschüssigem Formaldehyd aus der Reaktionsmischung, Umsetzung von einem Halogenacetylhalogenid mit der Reaktionsmischung und weitere Umsetzung der Reaktionsmischung mit einem Pyrazol der allgemeinen Formel

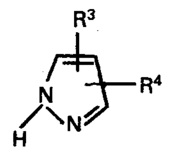

in welcher $R^3$ und $R^4$ die im Anspruch 1 angegebene Bedeutung haben, und einem Halogenwasserstoff bindenden Mittel.

8. Verfahren gemäss Anspruch 1, dadurch gekennzeichnet, dass man die Behandlung der Rohprodukte mit einer Säure erst nach der Abtrennung einer wässrigen Phase von den Rohprodukten vornimmt, wenn bei der Herstellung der Rohprodukte als Halogenwasserstoff bindendes Mittel eine wässrige Lösung einer Base verwendet worden ist.

9. Verfahren gemäss Anspruch 1, dadurch gekennzeichnet, dass die Reste R und $R^1$ in 2- und 6-Stellung zum Stickstoff stehen und jeweils Methyl bedeuten.

**Revendications**

1. Procédé de préparation, à partir des produits bruts correspondants, de composés pyrazoliques sensiblement purs de la formule générale

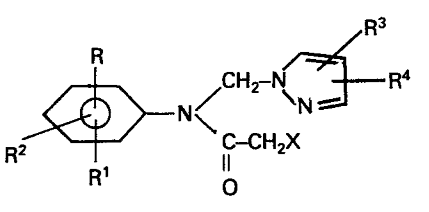

(I)

dans laquelle

R représente hydrogène, halogène, alkyle jusqu'à 5 atomes de carbone, alcoxy jusqu'à 3 atomes de carbone, perhalogénalkyle jusqu'à 3 atomes de carbone ou alcoxyalkyle jusqu'à 5 atomes de carbone,

$R^1$ représente hydrogène, halogène, alkyle jusqu'à 5 atomes de carbone, alcoxy jusqu'à 3 atomes de carbone, perhalogénalkyle jusqu'à 3 atomes de carbone ou alcoxyalkyle jusqu'à 5 atomes de carbone,

$R^2$ représente hydrogène, halogène, alkyle jusqu'à 5 atomes de carbone, alcoxy jusqu'à 3 atomes de carbone, perhalogénalkyle jusqu'à 3 atomes de carbone ou alcoxyalkyle jusqu'à 5 atomes de carbone, ou bien l'ensemble $R^2$ et R représente une chaîne alkylène ayant jusqu'à 6 atomes de carbone et reliée en position ortho et éventuellement substituée par des groupes alkyle ayant jusqu'à 4 atomes de carbone,

X représente chlore ou brome,

$R^3$ et $R^4$ sont identiques ou différents et représentent hydrogène, alkyle, alcoxy, alkylthio, carbalcoxy, perfluoroalkyle, chacun ayant jusqu'à 4 atomes de carbone, halogène, phényle, cyano ou carboxy, caractérisé par le fait que l'on traite les produits bruts avec des solutions aqueuses à 30 à 90% d'acides inorganiques forts, on sépare la solution aqueuse, on la dilue avec de l'eau et l'on sépare le produit brut ainsi obtenu du liquide aqueux.

2. Procédé selon la revendication 1, caractérisé par le fait que l'on utilise de l'acide chlorhydrique à 32 à 38%.

3. Procédé selon la revendication 1, caractérisé par le fait que l'on utilise de l'acide sulfurique à 40 à 80%.

4. Procédé selon la revendication 1, caractérisé par le fait que l'on extrait du liquide aqueux les produits purs obtenus avec un solvant organique approprié à leur traitement ultérieur.

5. Procédé selon la revendication 1, caractérisé par le fait que l'on utilise, comme produits de départ, des solutions de produits bruts dans un solvant organique non miscible ou peu miscible avec l'eau.

6. Procédé selon la revendication 1, caractérisé par le fait que l'on utilise, comme produits de départ, les produits bruts sous forme solide, finement divisée.

7. Procédé selon la revendication 1, caractérisé par le fait que l'on utilise, comme produits bruts, des produits que l'on obtient par réaction d'une aniline substituée de formule générale

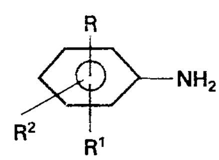

dans laquelle R, $R^1$ et $R^2$ ont la signification donnée à la revendication 1, avec du paraformaldéhyde dans un solvant organique inerte, on sépare par distillation l'eau et le formaldéhyde en excès du mélange de réaction, on fait réagir un halogénure d'acétylhalogène avec le mélange réactionnel et on fait réagir ensuite le mélange réactionnel avec du pyrazol de formule générale

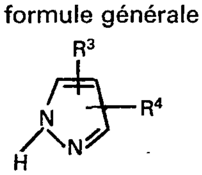

dans laquelle $R^3$ et $R^4$ ont la signification donnée à la revendication 1 et avec un moyen liant l'acide chlorhydrique.

8. Procédé selon la revendication 1, caractérisé par le fait que l'on n'effectue le traitement des produits bruts avec un acide qu'après séparation d'une phase gazeuse des produits bruts lorsque, pour la préparation des produits bruts, on a utilisé comme moyen liant l'acide chlorhydrique une solution aqueuse d'une base.

9. Procédé selon la revendication 1, caractérisé par le fait que les restes R et $R^1$ se trouvent en positions 2 et 6 par rapport à l'azote et représentent chacun méthyle.

**Claims**

1. A process for the preparation of substantially pure pyrazole compounds of the general formula

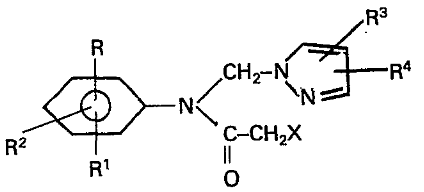

(I)

where

R is hydrogen, halogen, alkyl of up to 5 carbon atoms, alkoxy of up to 3 carbon atoms, perhaloalkyl of up to 3 carbon atoms or alkoxyalkyl of up to 5 carbon atoms,

$R^1$ is hydrogen, halogen, alkyl of up to 5 carbon atoms, alkoxy of up to 3 carbon atoms, perhaloalkyl of up to 3 carbon atoms or alkoxyalkyl of up to 5 carbon atoms,

$R^2$ is hydrogen, halogen, alkyl of up to 5 carbon atoms, alkoxy of up to 3 carbon atoms, perhaloalkyl of up to 3 carbon atoms or alkoxyalkyl of up to 5 carbon atoms, or $R^2$ together with R is an ortho-linked alkylene chain of up to 6 carbon atoms which is unsubstituted or substituted by alkyl of up to 4 carbon atoms,

X is chlorine or bromine, and

$R^3$ and $R^4$ are identical or different and each is hydrogen, alkyl, alkoxy, alkylthio, carbalkoxy or perfluoroalkyl, in each case of up to 4 carbon atoms, or halogen, phenyl, cyano or carboxyl, from the corresponding crude products, characterized in that the crude products are treated with 30 to 90% by weight aqueous solutions of strong acids, thereafter the aqueous solution is separated off and diluted with water, and the pure product which hereupon precipitates is separated from the aqueous fluid.

2. A process as claimed in claim 1, characterized in that hydrochloric acid of from 32 to 38% strength is used.

3. A process as claimed in claim 1, characterized in that sulphuric acid of from 40 to 80% strength is used.

4. A process as claimed in claim 1, characterized in that the precipitated pure product is extracted from the aqueous fluid by means of an organic solvent which is suitable for the further processing of the product.

5. A process as claimed in claim 1, characterized in that a solution of the crude product in a water-immiscible or only slightly water-miscible organic solvent is used as the starting material.

6. A process as claimed in claim 1, characterized in that the crude product, in a solid finely divided form, is used as the starting material.

7. A process as claimed in claim 1, characterized in that a product is used as crude product which is obtained by reacting a substituted aniline of the general formula

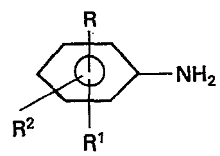

where R, $R^1$ and $R^2$ have the meanings given in claim 1, with paraformaldehyde in an inert organic solvent, distilling off water and excess formaldehyde from the reaction mixture, reacting the mixture with a haloacetyl halide and then reacting it further with a pyrazole of the general formula

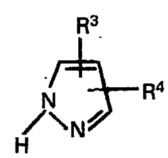

where $R^3$ and $R^4$ have the meanings given in claim 1 and an agent which binds hydrogen halide.

8. A process as claimed in claim 1, characterized in that, if an aqueous solution of a base has been used as the agent which binds hydrogen halide during the preparation of the crude product, the treatment of the crude product with an acid is only carried out after the crude product has been separated from an aqueous phase.

9. A process as claimed in claim 1, characterized in that the radicals R and $R^1$ are in the 2- and 6-positions to the nitrogen and each is methyl.